# EUROPEAN PATENT APPLICATION

(11) **EP 4 325 546 A1**
(43) Date of publication of application: **21.02.2024**
(21) Application number: 22788082.0
(22) Date of filing: 31.03.2022
(51) Int. Cl.: H01J 65/00, A61L 2/10

(54) **ULTRAVIOLET LIGHT IRRADIATION DEVICE**

(30) Priority: 14.04.2021 JP 2021068405; 18.02.2022 JP 2022023675
(71) Applicant: Ushio Denki Kabushiki Kaisha, Tokyo 100-8150 (JP)
(72) Inventor: KUNO,Akihiro, Tokyo 100-8150 (JP); YAGYU,Hideaki, Tokyo 100-8150 (JP); FUJISAWA,Shigeki, Tokyo 100-8150 (JP)
(74) Representative: Maiwald GmbH
(86) International application number: PCT/JP2022/016712
(87) International publication number: WO 2022/220157

(57) **Abstract**

To provide an ultraviolet light irradiation device capable of inactivating bacteria, viruses, and the like with increased efficiency while ensuring safety. An ultraviolet light irradiation device according to the present invention includes a light source to emit ultraviolet light having a wavelength within a range of 190 nm or more and less than 240 nm, a housing to house the light source, a light extraction part to extract the ultraviolet light emitted from the light source out of the housing, and a diffusion transmission member to diffuse and transmit the ultraviolet light.

## Description

### TECHNICAL FIELD

The present invention relates to an ultraviolet light irradiation device.

### BACKGROUND ART

Conventionally, technologies for inactivating bacteria or viruses by irradiating bacteria or viruses with ultraviolet light have been known. DNA exhibits the highest absorption characteristics around a wavelength of 260 nm, and therefore in many cases, ultraviolet light that is emitted from a light source such as a low-pressure mercury lamp and has a wavelength of around 254 nm is used. Methods for inactivating bacteria or viruses by ultraviolet light are characterized by being able to sterilize a target space or a target object only by irradiating the target space or the target object with ultraviolet light without spraying chemicals or the like.

For instance, Patent Document 1 below describes a lighting fixture that includes a built-in germicidal lamp made up of a low-pressure mercury lamp and that is installed at a kitchen or other places to emit ultraviolet light having a wavelength of 254 nm and perform sterilization treatment.

Patent Document 2 below describes a sterilization apparatus that performs sterilization treatment by irradiating bacteria and viruses floating in a room with ultraviolet light.

The ultraviolet light irradiation devices described in Patent Documents 1 and 2 below use ultraviolet light in a wavelength band hazardous to the human body. Hence, these ultraviolet light irradiation devices are provided with measures such as controlling a direction in which the ultraviolet light is emitted to prevent the human body from being irradiated with the ultraviolet light.

### PRIOR ART DOCUMENT

### PATENT DOCUMENTS

Patent Document 1: JP-U-63-187221
Patent Document 2: JP-A-2017-018442

### SUMMARY OF THE INVENTION

### PROBLEMS TO BE SOLVED BY THE INVENTION

However, a relatively high number of bacteria, fungi, and viruses, or the like are present particularly on the surfaces of human bodies (e.g., skins and hairs) and the surfaces of objects that humans frequently touch (e.g., furniture and office equipment). In spaces where humans are coming and going, bacteria, viruses, or the like (hereinafter may be collectively referred to as "pathogens") attached to airborne droplets or aerosols released from humans or animals and spread through expired air or saliva or by coughing or sneezing are present in many cases.

Conventional ultraviolet light irradiation devices such as those described in Patent Documents 1 and 2 above are not allowed to irradiate regions or spaces where a relatively high number of bacteria, viruses, or the like are likely to be present, such as the surfaces of human bodies and spaces where humans are frequently coming and going, with ultraviolet light because of concern about an influence of ultraviolet light irradiation on the human body. Thus, when a human is present in a space, the space is irradiated while exposure of the human to radiation is always avoided. This prevents inactivation from being efficiently performed. In a space where a human is moving around, it is difficult to reliably control a device such that the human is not irradiated. Thus, even a device that is controlled such that exposure of humans to radiation is avoided carries not a little risk associated with safety.

In light of the above problems, it is an object of the present invention to provide an ultraviolet light irradiation device capable of inactivating bacteria, viruses, and the like with increased efficiency while ensuring safety.

### MEANS FOR SOLVING THE PROBLEMS

An ultraviolet light irradiation device according to the present invention includes:
a light source to emit ultraviolet light having a wavelength within a range of 190 nm or more and less than 240 nm;
a housing to house the light source;
a light extraction part to extract the ultraviolet light emitted from the light source out of the housing; and
a diffusion transmission member to diffuse and transmit the ultraviolet light.

In the present specification, a member configured to "diffuse and transmit" ultraviolet light is a member that converts ultraviolet light incident on the member and transmits the incident ultraviolet light such that an emission angle of the ultraviolet light emitted from the member is greater than an incident angle of the incident ultraviolet light. In the present specification, a member that displays a property in such a way that ultraviolet light is "diffused and transmitted" through the member is referred to as a "diffusion transmission member". Examples of the diffusion transmission member include a thin film-shaped member laminated on a surface of an object and a sheet-shaped member.

In recent years, there have been many reports indicating that ultraviolet light having a wavelength within a range of 190 nm or more and less than 240 nm is effective in inactivating pathogens while having an extremely small influence on the human body. Inactivation by ultraviolet light in the wavelength range, in particular, has attracted attention due to the recent novel coronavirus (Covid-19) epidemic. For instance, ultraviolet light having a wavelength of 240 nm or more is readily transmitted through human skin, and readily permeates through an inside of skin. Therefore, cells inside human skin are easily damaged. In contrast, ultraviolet light having a wavelength of less than 240 nm is apt to be absorbed on a surface (for example, stratum corneum) of human skin, and ultraviolet light having a shorter wavelength is less apt to permeate through an inside of skin. Thus, such ultraviolet light has a small influence on the human body. This is also proved by an ultraviolet light absorption spectrum of protein constituting skin in which light absorptance is high at wavelengths less than 240 nm. In particular, safety to the human body can be improved in a band of shorter wavelengths. The ultraviolet light is desirably in a wavelength band of 237 nm or less and more desirably in a wavelength band of 235 nm or less, for example. Since short-wavelength light is apt to generate ozone in a space, ultraviolet light in a wavelength band of 200 nm or more is more suitable. From this point of view, it is desirable to actively use ultraviolet light in a wavelength band of 200 nm or more and 235 nm or less.

However, although ultraviolet light with wavelengths of 190 nm or more and less than 240 nm has an extremely small influence on the human body compared to ultraviolet light emitted from low-pressure mercury lamps, regulation values concerning an integrated irradiation dose to the human body are prescribed in consideration of safety. At the time of applying the present specification for a patent, it is recommended to set the integrated irradiation dose of ultraviolet light radiated to the human body to be within the regulation value (a tolerance limit value) defined by the American Conference of Governmental Industrial Hygienists (ACGIH). For instance, the tolerance limit value for the integrated irradiation dose of ultraviolet light with a wavelength of 222 nm a day (eight hours) is specified to be 22 mJ/cm². In the present specification, the tolerance limit value is a current numerical value and is a numerical value that may be altered in the future.

In view of the above circumstances, an ultraviolet light irradiation device that is expected to be used in a space where a human is present preferably irradiates the space across a wide area with ultraviolet light so as to comply with the tolerance limit value rather than irradiating a localized part of the space with ultraviolet light at high irradiance. Specifically, when a dose of ultraviolet irradiation is set so as not to exceed the tolerance limit value, it is desirable in terms of managing the dose of ultraviolet irradiation to set an upper limit on the dose of irradiation so as to meet a level of high-intensity light locally received in a region. Unfortunately, in this case, the dose of ultraviolet irradiation needs to be restricted more than necessary in a region where irradiance is low relative to a region where irradiance is high. Hence, the inventors of the present invention have studied an ultraviolet light irradiation device that includes a member (a diffusion transmission member) configured to diffuse and transmit ultraviolet light to allow ultraviolet light emitted from a light source to be radiated across a wide area.

The ultraviolet light irradiation device configured as described above is able to irradiate a space across a wide area with ultraviolet light rather than irradiating a localized part of the space with ultraviolet light at high irradiance.

In the ultraviolet light irradiation device, a thickness of the diffusion transmission member may be less than 1.5 mm.

With an increase in thickness of the diffusion transmission member, ultraviolet light is apt to be refracted (dispersed) inside the diffusion transmission member. When the thickness exceeds a certain level, ultraviolet light incident on the diffusion transmission member is perfectly diffused. The ultraviolet light that is perfectly diffused is in a state in which brightness of the light is constant on an emission surface of the diffusion transmission member irrespective of a direction in which the light is emitted and a flux of the emitted light on the emission surface is proportional to cosθ (θ is an emission angle of the light emitted from the emission surface). With an increase in wavelength of the incident ultraviolet light, a threshold value of the thickness of the diffusion transmission member at which the light is perfectly diffused rises. The diffusion transmission member can diffuse ultraviolet light even when the ultraviolet light is not perfectly diffused. Thus, the diffusion transmission member is not necessarily required to have a thickness at which the light is perfectly diffused. However, if ultraviolet light is desired to be radiated across a wider area, the diffusion transmission member preferably has a thickness at which incident ultraviolet light is perfectly diffused.

When the diffusion transmission member gets thicker, frequency with which ultraviolet light repeats refraction (dispersion) increases and ultraviolet light energy is apt to be expended inside the member. As a result, ultraviolet light is less apt to be extracted, and average transmittance to ultraviolet light decreases. When the thickness of the diffusion transmission member remains the same to make a comparison, the average transmittance decreases with a decrease in wavelength of the incident ultraviolet light. Thus, it is feared that the diffusion transmission member that is made thicker than necessary may cause ultraviolet light in a wavelength band used for inactivation of pathogens as well as ultraviolet light hazardous to the human body to be less apt to be transmitted, reducing an inactivating effect.

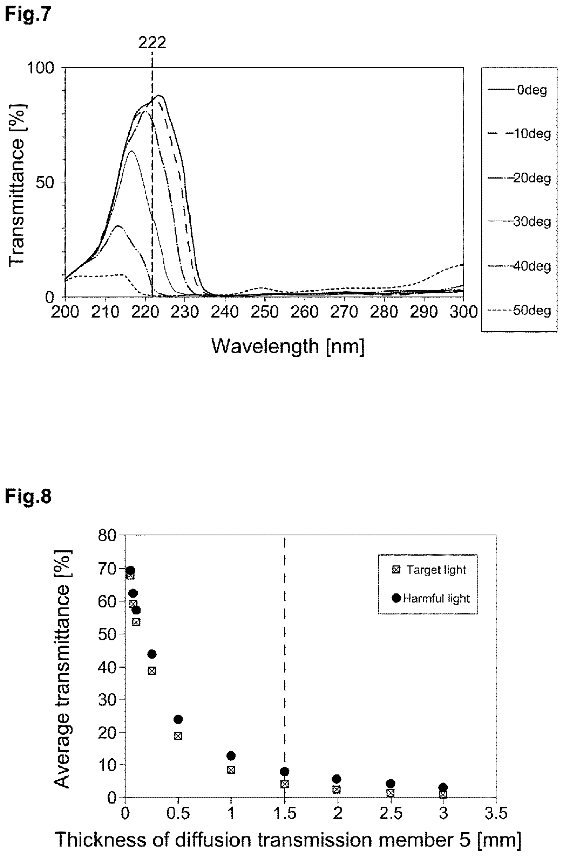

The inventors of the present invention with the intension of finding a suitable range of the thickness of the diffusion transmission member have conducted a verification experiment to ascertain a relationship between the thickness of the diffusion transmission member and the average transmittance to ultraviolet light. According to the verification experiment, it has been ascertained that the average transmittance to ultraviolet light in a wavelength band used for inactivation is satisfactorily high when the thickness of the diffusion transmission member is less than 1.5 mm. The verification experiment will be described in detail with reference to Fig. 7 in "MODE FOR CARRYING OUT THE INVENTION".

Thus, the ultraviolet light irradiation device is able to irradiate a wider area with ultraviolet light having a wavelength within a range of 190 nm or more and less than 240 nm, which has an extremely small influence on the human body while suppressing a decrease in average transmittance to the ultraviolet light in the wavelength band and ensuring safety.

The ultraviolet light irradiation device may further include an optical filter to transmit at least part of ultraviolet light having a wavelength within a range of 190 nm or more and less than 240 nm and suppress transmission of ultraviolet light having a wavelength within a range of 240 nm or more and less than 300 nm.

In the present specification, "suppressing transmission of ultraviolet light" refers to decreasing a ratio of the intensity of ultraviolet light having a wavelength of 240 nm or more and less than 300 nm out of ultraviolet light transmitted through an optical filter relative to light intensity at a peak wavelength in a wavelength range of 190 nm or more and less than 240 nm out of ultraviolet light emitted from an ultraviolet light source. In the present invention, by using the optical filter, the ultraviolet light irradiation device substantially suppresses radiation intensity of ultraviolet light that is radiated to outside the device and that has a wavelength of 240 nm or more and less than 300 nm.

The ultraviolet light irradiation device configured as described above substantially suppresses the intensity of ultraviolet light having a wavelength within a range of 240 nm or more and less than 300 nm, which is hazardous to the human body, out of ultraviolet light emitted from the device. In other words, an ultraviolet light irradiation device with improved safety is achieved. For instance, the ultraviolet light irradiation device may be configured to essentially prevent transmission of ultraviolet light having a wavelength within a range of 240 nm or more and less than 300 nm. "Essentially preventing transmission of ultraviolet light" described herein refers to suppressing the intensity of ultraviolet light in the wavelength range to 5% or less of the intensity at the peak wavelength, concerning ultraviolet light that is incident at an incident angle of 0° and emitted at an emission angle of 0°. The intensity of ultraviolet light in the wavelength band suppressed by the optical filter is preferably suppressed to 2% or less and more preferably suppressed to 1% or less relative to the intensity at the peak wavelength.

In the ultraviolet light irradiation device, the diffusion transmission member may be configured such that ultraviolet light transmitted through the optical filter is incident on the diffusion transmission member.

An emission angle at which ultraviolet light is emitted from the diffusion transmission member is greater than an incident angle at which the ultraviolet light is incident on the diffusion transmission member. In other words, the angle of ultraviolet light emitted from the diffusion transmission member is wider compared to the ultraviolet light before being incident on the diffusion transmission member. Thus, it is difficult to get the ultraviolet light emitted from the member to be incident on a specific region. To put it another way, to ensure that ultraviolet light is incident on the optical filter with increased reliability, ultraviolet light to be incident on an incident surface of the optical filter is preferably ultraviolet light in a state before being incident on the diffusion transmission member.

As described later with reference to Fig. 7, a transmission spectrum of the optical filter changes in response to an incident angle at which the light is incident on the incident surface of the optical filter and transmittance to ultraviolet light in the wavelength band that is to be transmitted through decreases with an increase in incident angle. Thus, ultraviolet light incident on the optical filter should have a high percentage of a light beam component that is incident on the incident surface of the optical filter at a narrow incident angle (low angle).

Moreover, ultraviolet light transmitted through the diffusion transmission member and incident on the optical filter has a high percentage of a light beam component that is incident on the incident surface of the optical filter at a wide incident angle compared to a case in which ultraviolet light is directly incident on the optical filter without being transmitted through the diffusion transmission member. As described above, transmittance of the optical filter to ultraviolet light in the wavelength band that is to be transmitted through characteristically decreases with an increase in incident angle. Thus, an increase in the percentage of the light beam component that is incident on the incident surface of the optical filter at a wide incident angle leads to a decrease in the percentage of the light beam component that is transmitted through the optical filter.

In other words, a case in which ultraviolet light transmitted through the diffusion transmission member is incident on the optical filter leads to an increase of the light beam component that is not transmitted through the optical filter, and thus the ultraviolet light irradiation device is preferably configured to get ultraviolet light to be transmitted through the optical filter and be incident on the diffusion transmission member.

The ultraviolet light irradiation device may include:
a base material having a plate shape and including a first principal surface disposed adjacent to the light source, the base material being configured to transmit ultraviolet light having a wavelength within a range of 190 nm or more and less than 240 nm; and
an optical filter disposed on a second principal surface of the base material opposite the first principal surface, the optical filter being configured to transmit at least part of ultraviolet light having a wavelength within a range of 190 nm or more and less than 240 nm and suppress transmission of ultraviolet light having a wavelength within a range of 240 nm or more and less than 300 nm, and
the diffusion transmission member may be configured to diffuse and transmit ultraviolet light emitted from the optical filter.

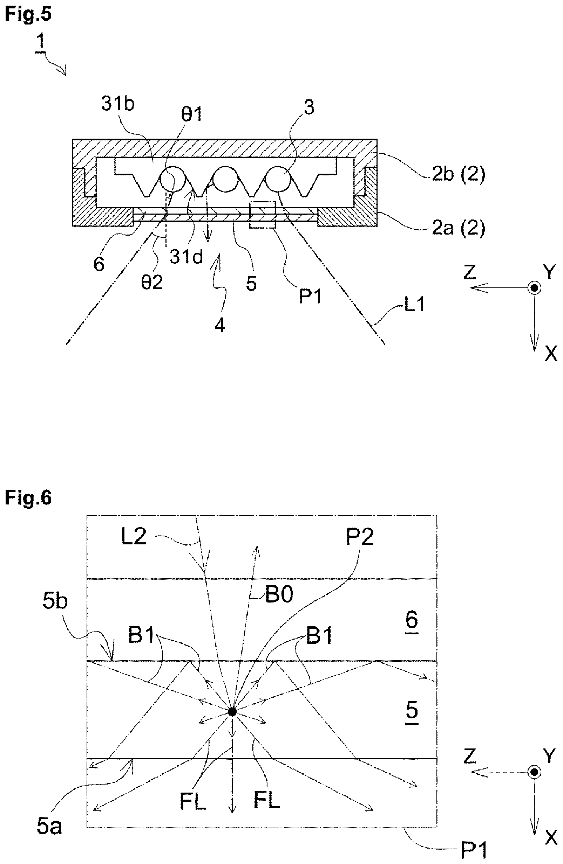

Ultraviolet light transmitted through the optical filter and incident on the diffusion transmission member is refracted or reflected inside the diffusion transmission member, and a part of the light is diffused and transmitted and an other part is returned to an optical filter side (see Fig. 6). In the present specification, the light returned from the diffusion transmission member to the optical filter side is sometimes referred to as "return light".

This return light contains ultraviolet light in the wavelength band (190 nm or more and less than 240 nm) useful for inactivation of pathogens. Hence, the inventors of the present invention have studied a way of achieving efficient inactivation by getting at least part of the return light to be reflected again to a diffusion transmission member side and utilizing the reflected light for inactivation.

The optical filter transmits ultraviolet light having a wavelength within a range of 190 nm or more and less than 240 nm but reflects part of the ultraviolet light. This is because optical filters with a transmittance of 100% do not exist and many optical filters are characterized by varying transmittance to ultraviolet light depending on the incident angle. For instance, regarding an optical filter made from a dielectric multilayer film, transmittance to ultraviolet light having a wavelength within a range of 190 nm or more and less than 240 nm decreases and reflectance to such ultraviolet light increases with an increase in incident angle of the ultraviolet light (see Fig. 7).

In other words, the ultraviolet light irradiation device includes the diffusion transmission member and the optical filter and thus is able to utilize part of the return light for inactivation.

However, the inventors of the present invention have noticed that simply being equipped with the diffusion transmission member and the optical filter, the ultraviolet light irradiation device may not be able to satisfactorily improve light usage efficiency since the intensity of the return light is substantially attenuated depending on a positional relationship between these parts. This will be described below with reference to the drawings.

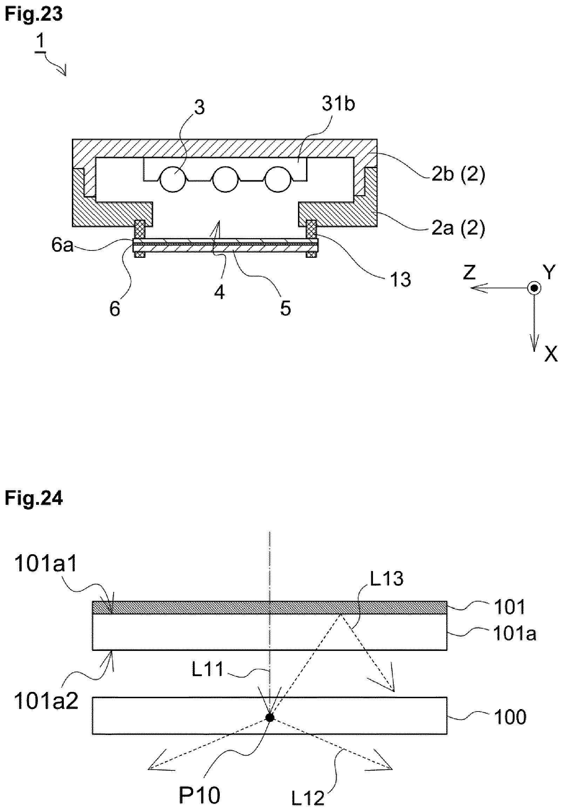

Fig. 24 is a schematic view showing an example disposition of a diffusion transmission member 100, an optical filter 101, and a base material 101a to which the optical filter 101 is fixed. Here, let us study a configuration, as shown in Fig. 24, in which ultraviolet light L10 emitted from a light source (not shown) is incident on the optical filter 101 disposed on a first principal surface 101a1 of the base material 101a and then is emitted from a second principal surface 101a2 to be incident on the diffusion transmission member 100.

It is assumed that ultraviolet light L11 incident on the diffusion transmission member 100 is isotropically diffused at a point P10 in the diffusion transmission member 100. Consequently, a part of the ultraviolet light diffused inside the diffusion transmission member 100 is emitted from a principal surface of the diffusion transmission member opposite the base material 101a (ultraviolet light L12), and an other part travels toward the base material 101a (return light L13).

As observed from Fig. 24, the return light L13 is incident on the second principal surface 101a2 of the base material 101a and then travels inside the base material 101a to reach the optical filter 101. A part of the return light L13 is reflected off the optical filter 101 toward the diffusion transmission member 100. The return light L13 reflected off the optical filter 101 travels again inside the base material 101a, is emitted from the second principal surface 101a2, and then is incident on the diffusion transmission member 100.

As described above, if the configuration shown in Fig. 24 is adopted, the return light L13 emitted from the diffusion transmission member 100 toward the optical filter 101 moves back and forth between the first principal surface 101a1 and the second principal surface 101a2 of the base material 101a while being reflected off the optical filter 101 and being incident again on the diffusion transmission member 100.

Although the base material 101a is made of a material that displays transparency to ultraviolet light having a wavelength within a range of 190 nm or more and less than 240 nm, which is used for inactivation, materials with a transmittance of 100% do not exist. In general, ultraviolet light is more apt to be absorbed in a case in which the light travels inside a base material compared to a case in which the light travels through the air. In particular, a difference in absorptance between the cases is more noticeable for ultraviolet light in the wavelength range described above.

A part of the return light L13 that is incident again on the diffusion transmission member 100 is diffused and reflected by the diffusion transmission member 100 to travel again toward the base material 101a. Thus, in some cases, the return light L13 is repeatedly diffused by the diffusion transmission member 100 and reflected from the optical filter 101, and disappears before being emitted toward a region subject to inactivation.

As a result, the configuration shown in Fig. 24 enables reuse of return light but does not satisfactorily improve light usage efficiency since the intensity of the return light L13 is substantially attenuated through the base material 101a.

In contrast, an ultraviolet light irradiation device according to the present invention has a configuration in which the optical filter 101 is disposed on the second principal surface 101a2 of the base material 101a. Thus, return light reaches the optical filter without passing through an inside of the base material. This suppresses attenuation of the intensity of the return light through the base material. In other words, the ultraviolet light irradiation device configured as described above makes it possible to emit the return light with high intensity maintained. To put it another way, a highly efficient ultraviolet light irradiation device is achieved.

In the ultraviolet light irradiation device, the diffusion transmission member and the optical filter may be put into contact with each other.

The above configuration enables return light being returned to the optical filter side by the diffusion transmission member to be incident on the optical filter without traveling through the air or the like. In other words, according to the above configuration, the attenuation of the intensity of the return light, which takes place between the diffusion transmission member and the optical filter, is suppressed, and this facilitates emission of return light with high intensity maintained toward a region or an object subject to inactivation.

The ultraviolet light irradiation device may include a holding member to hold the diffusion transmission member.

The above configuration enables any of a particle sintered body, a filmy member made of a resin material, and a sheet-shaped diffusion transmission member that are difficult to be fixed in isolation form to be fixed to a part such as the housing.

Moreover, in the ultraviolet light irradiation device, the holding member may be a member that transmits at least part of ultraviolet light having a wavelength within a range of 190 nm or more and less than 240 nm.

In the ultraviolet light irradiation device, the diffusion transmission member may be detachable from the housing.

The above configuration enables the diffusion transmission member alone to be readily replaced when deterioration of the diffusion transmission member is confirmed or when a lighting operation is performed for a predetermined time. In other words, maintenance of the ultraviolet light irradiation device is simplified. In particular, in the configuration including the optical filter, in which the diffusion transmission member is disposed downstream of the optical filter on an optical path, ultraviolet light in a state before being incident on the diffusion transmission member can be guided to the optical filter and replacement of the diffusion transmission member can be simplified.

In the ultraviolet light irradiation device, a main ingredient of the diffusion transmission member may be a fluorine-based resin, a polyethylene-based resin, or a polyester-based resin.

Examples of the fluorine-based resin include polytetrafluoroethylene (PTFE), 4-fluoroethylene-perchloroalkoxy copolymers (PFA), 4-fluoroethylene-6-fluoropropylene copolymers (FEP), 2-ethylene-4-fluoroethylene copolymers (ETFE), poly(3-fluorochloroethylene) (PCTFE), polyvinylidene fluoride (PVDF), and polyvinyl fluoride (PVF). Among these options, PTFE in particular is preferable in view of resistance to ultraviolet light, cost effectiveness, and other conditions.

Examples of the polyethylene-based resin include but not limited to low-density polyethylene, medium-density polyethylene, high-density polyethylene, and linear low-density polyethylene.

Examples of the polyester-based resin include but not limited to polyethylene terephthalate (PET), polyethylene naphthalate (PEN), polyethylene naphthalate (PEN), polybutylene terephthalate (PBT), and polycyclohexylenedimethylene terephthalate (PCT). Among these options, PET in particular is preferable in view of resistance to ultraviolet light, cost effectiveness, and other conditions.

In the ultraviolet light irradiation device, a main ingredient of the diffusion transmission member may be a ceramic material.

Moreover, in the ultraviolet light irradiation device, the main ingredient of the diffusion transmission member may be silica or alumina.

Moreover, in the ultraviolet light irradiation device, average transmittance of the diffusion transmission member to ultraviolet light having a wavelength within a range of 190 nm or more and less than 240 nm is preferably 10% or more.

In the present specification, a "main ingredient" of a member refers to a material whose content by percentage is highest among materials that the member is made of.

A product covered by the present invention can provide sterilization and virus inactivation performance intrinsic to ultraviolet light without causing erythema or keratitis on the skin or eyes of a human or an animal. In particular, taking advantage of a characteristic of being able to be used in an environment where humans are present in contrast to conventional ultraviolet light sources, the product can be installed in such an environment indoors or outdoors to irradiate the entire environment and provide virus inhibition and bacteria elimination in the air and on a surface of parts installed in the environment.

This accords with Goal 3 "Ensure healthy lives and promote well-being for all at all ages" included in the Sustainable Development Goals (SDGs) led by the United Nations, and will greatly contribute to the goal target 3.3 "By 2030, end the epidemics of AIDS, tuberculosis, malaria and neglected tropical diseases and combat hepatitis, water-borne diseases and other communicable diseases".

### EFFECT OF THE INVENTION

According to the present invention, it is possible to achieve an ultraviolet light irradiation device capable of inactivating bacteria, viruses, and the like with increased efficiency while ensuring safety.

### BRIEF DESCRIPTION OF THE DRAWINGS

Fig. 1 is a schematic view showing an external appearance of an ultraviolet light irradiation device according to an embodiment.
Fig. 2 is a schematic view showing an external appearance of an ultraviolet light irradiation device according to an embodiment.
Fig. 3 is a drawing showing a configuration of a light source.
Fig. 4 is a drawing showing a state of the light source in Fig. 3 from which a light-emitting tube is removed.
Fig. 5 is a cross-sectional view of an ultraviolet light irradiation device according to an embodiment, viewed along an Y direction.
Fig. 6 is an enlarged view of a region P1 in Fig. 5.
Fig. 7 is a graph showing an example of transmission spectra of an optical filter by incident angle.
Fig. 8 is a graph showing a relationship between a thickness of a diffusion transmission member and average transmittance.
Fig. 9 is a graph showing a relationship between a thickness of a diffusion transmission member and a ratio of average transmittance to target light to average transmittance to harmful light.
Fig. 10 is a cross-sectional view of an ultraviolet light irradiation device according to an embodiment, viewed along the Y direction.
Fig. 11 is an enlarged view of a region P1 in Fig. 10.
Fig. 12 is a schematic view showing a configuration of an example in a verification experiment.
Fig. 13 is a schematic view showing an external appearance of an ultraviolet light irradiation device according to an embodiment.
Fig. 14 is a cross-sectional view of an ultraviolet light irradiation device according to an embodiment, viewed along an Y direction.
Fig. 15 is a cross-sectional view of an ultraviolet light irradiation device according to an embodiment, viewed along an Y direction.
Fig. 16 is a schematic view showing an external appearance of an ultraviolet light irradiation device according to an embodiment.
Fig. 17 is a cross-sectional view of an ultraviolet light irradiation device according to an embodiment, viewed along an Y direction.
Fig. 18 is a cross-sectional view of an ultraviolet light irradiation device according to an embodiment, viewed along an Y direction.
Fig. 19 is a cross-sectional view of an ultraviolet light irradiation device according to another embodiment, viewed along the Y direction.
Fig. 20 is a cross-sectional view of an ultraviolet light irradiation device according to another embodiment, viewed along the Y direction.
Fig. 21 is a cross-sectional view of an ultraviolet light irradiation device according to another embodiment, viewed along the Y direction.
Fig. 22 is a cross-sectional view of an ultraviolet light irradiation device according to another embodiment, viewed along the Y direction.
Fig. 23 is a cross-sectional view of an ultraviolet light irradiation device according to another embodiment, viewed along the Y direction.
Fig. 24 is a schematic view showing an example disposition of a diffusion transmission member, an optical filter, and a base material.

### MODES FOR CARRYING OUT THE INVENTION

An ultraviolet light irradiation device according to the present invention will be described hereinafter with reference to the drawings. It should be noted that the drawings referred to below regarding the ultraviolet light irradiation device are all schematic illustrations and dimensional ratios and numbers of parts on the drawings do not necessarily match the actual dimensional ratios and numbers of parts.

### [First embodiment]

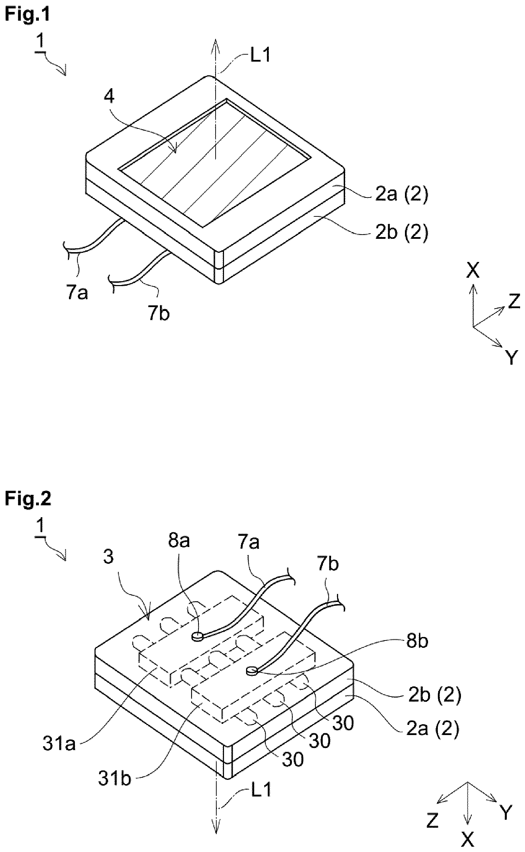

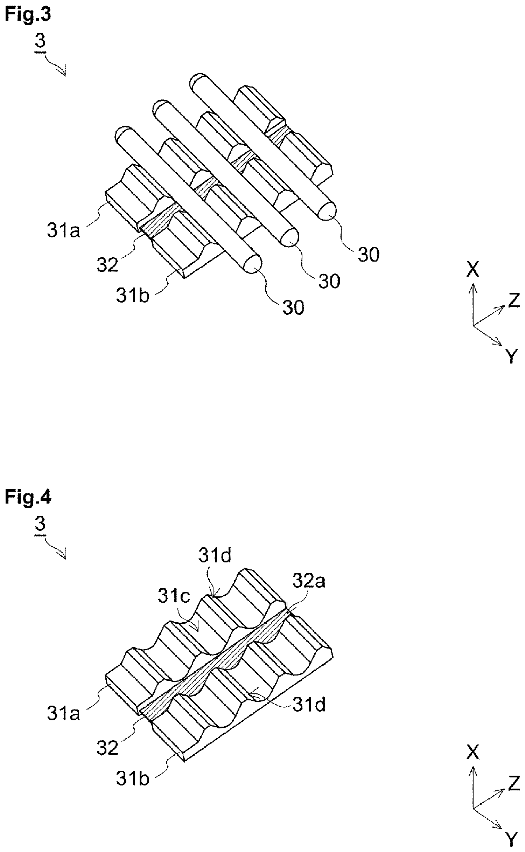

Figs. 1 and 2 are schematic views each showing an external appearance of an ultraviolet light irradiation device 1 according to a first embodiment. Fig. 3 is a drawing showing a configuration of a light source 3. As shown in Fig. 2, the ultraviolet light irradiation device 1 of the first embodiment includes a housing 2 and the light source 3 housed inside the housing 2.

In the description given hereinafter, as shown in Fig. 3, a direction in which a plurality of light-emitting tubes 30, which are described later and included in the light source 3, are arranged is defined as a Z direction. A direction in which the light-emitting tubes 30 are extending is a Y direction, and a direction orthogonal to both the Y direction and the Z direction is an X direction.

Furthermore, positive and negative orientations distinguished from each other for directional expression will be described as a "+X direction" and a "-X direction" by adding positive and negative signs, while a direction expressed without distinction between positive and negative orientations will be described simply as the "X direction". For the ultraviolet light irradiation device 1 shown in Figs. 1 and 2, a direction in which ultraviolet light is extracted corresponds to "+X direction".

The ultraviolet light irradiation device 1 of the first embodiment, as shown in Figs. 1 and 2, includes the housing 2, the light source 3, and a pair of feeders (7a, 7b).

As shown in Fig. 2, the housing 2 includes a cover member 2a and a main body 2b. The cover member 2a has a light extraction part 4 through which ultraviolet light generated by the light source 3 housed inside is extracted out. The main body 2b has a pair of feeding terminals (8a, 8b) that are connected to an external power source (not shown) via the respective feeders (7a, 7b).

The housing 2 is made up of the cover member 2a and the main body 2b that are combined together to house the light source 3 inside. However, the cover member 2a and the main body 2b may be integrated by being joined together by a rotational member, for example.

The peak wavelength for a KrCI excimer lamp is intended to include a difference among individual excimer lamp products and permit not only absolutely precise 222.0 nm but also a wavelength error of within ±3.0 nm from the reference point, 222.0 nm. Similar considerations apply to the KrBr excimer lamp.

In the first embodiment, the light extraction part 4 is a light exit window made of quartz glass. Superimposition of the light exit window, an optical filter 6 and a diffusion transmission member 5, which are described later, forms the light extraction part. Ultraviolet light emitted from the light source 3 passes through the optical filter 6 and the diffusion transmission member 5 forming the light extraction part 4 and is emitted as ultraviolet light L1 out of the housing 2.

In the first embodiment, the light source 3, as shown in Fig. 3, is an excimer lamp that includes a pair of electrodes (31a, 31b) and the light-emitting tubes 30. In the excimer lamp included in the ultraviolet light irradiation device 1 of the first embodiment, krypton (Kr) gas and chlorine (Chlorine) gas are sealed in the light-emitting tubes 30. When a voltage is applied between the electrodes (31a, 31b), ultraviolet light having a main emission wavelength of 222 nm is generated inside the light-emitting tubes 30.

The "main emission wavelength" described herein indicates, when a wavelength range Z(λ) covering ±10 nm of a wavelength A is defined in an emission spectrum, a wavelength λi in the wavelength range Z(Ai) at which an integrated intensity accounts for 40% or more of a total integrated intensity of the emission spectrum.

The light source 3 may be an excimer lamp that is configured differently from the one described above, as long as the excimer lamp is a light source that emits ultraviolet light having a main emission wavelength within a range of 190 nm or more and less than 240 nm. The light source may be a light source that emits ultraviolet light having a peak wavelength within a range of 190 nm or more and less than 240 nm. For instance, an excimer lamp that may be adopted is an excimer lamp that includes krypton (Kr) gas and bromine (Br) gas sealed in a light-emitting tube 30 and that emits ultraviolet light having a main emission wavelength of 207 nm. The light source 3 is not limited to excimer lamps, and a light source such as an LED may be adopted. An AIGaN-based LED or an MgZnO-based LED having a main emission wavelength of less than 240 nm can be adopted, for example.

In a case of using a coherent light source as the light source 3, the coherent light source may emit coherent ultraviolet light from a gas laser or a solid laser element, or the coherent light source may include a wavelength conversion element to newly generate coherent light having different wavelengths using light emitted from a gas laser or a solid laser element. The wavelength conversion element that may be used, for example, is a non-linear optical crystal that multiplies the frequency of light emitted from a laser element to generate high-order harmonic waves such as second harmonic generation (SHG) waves or third harmonic generation (THG) waves.

The pair of the electrodes (31a, 31b) are fixed to the main body 2b and are electrically connected to the respective connection terminals (8a, 8b). With this configuration, the connection terminals (8a, 8b) are electrically connected to an external power source (not shown) via the feeders (7a, 7b).

Fig. 4 is a drawing showing a state of the light source in Fig. 3 from which the light-emitting tubes 30 are removed. As shown in Figs. 3 and 4, the electrodes (31a, 31b) are arranged so as to be separated from each other in the Y direction and are block-shaped members that have recesses on which the light-emitting tubes 30 are placed and that are each made of a conductive material. The electrodes (31a, 31b) each have a tapered surface 31d to efficiently guide ultraviolet light generated by the light-emitting tubes 30 to the light extraction part 4.

A specific material for the electrodes (31a, 31b) may be Al, an AI alloy, or stainless steel, for example. In the first embodiment, the material for the electrodes (31a, 31b) is Al, and an entire surface on a +X side of the electrodes constitutes a first reflecting surface 31c. Light being generated in the light-emitting tubes 30 and traveling toward a side opposite the light extraction part 4 (a -X side) is reflected off the first reflecting surface to travel toward the light extraction part 4 (the +X side).

In the first embodiment, as shown in Fig. 4, a reflecting member 32 is disposed between the electrodes (31a, 31b) to constitute a second reflecting surface 32a that reflects light traveling toward the -X side to get the light to travel toward the +X side.

A configuration of the light extraction part 4 included in the housing 2 will be described in detail below. Fig. 5 is a cross-sectional view of the ultraviolet light irradiation device 1 according to the first embodiment, viewed along the Y direction. Fig. 6 is an enlarged view of a region P1 in Fig. 5. As shown in Fig. 5, the light extraction part 4 includes the optical filter 6 and the diffusion transmission member 5 formed on a principal surface of the optical filter 6.

The optical filter 6 may be a dielectric multilayer film including laminated layers with different refractive indices. The optical filter is, for example, a dielectric multilayer film including laminated layers of silica (SiO₂) and hafnia (HfO₂) with different refractive indices. In the first embodiment, the dielectric multilayer film is configured to transmit ultraviolet light having a wavelength within a range of 190 nm or more and less than 240 nm and essentially prevent transmission of ultraviolet light having a wavelength of 240 nm or more and less than 300 nm by adjusting a film thickness of each layer and a number of the layers. This feature will be described later with reference to Fig. 7. The optical filter 6 may be made of a material, such as alumina (Al₂O₃) and zirconia (ZrO₂), other than silica and hafnia.

If the light source 3 is a light source that hardly emits ultraviolet light having a wavelength component within a range of 240 nm or more and less than 300 nm due to a narrow intensity spectrum width, the ultraviolet light irradiation device 1 may not include the optical filter 6.

In the first embodiment, the diffusion transmission member 5 is a plate-shaped member having PTFE as a main ingredient and a thickness of 1.0 mm and is disposed such that ultraviolet light emitted from the optical filter 6 is incident on the diffusion transmission member. The diffusion transmission member 5, as shown in Fig. 5, diffuses and transmits incident ultraviolet light such that an emission angle θ2 is greater than an incident angle θ1. In Fig. 5, an appearance of a principal ray of the traveling ultraviolet light is schematically indicated by a two-dot chain line.

Ultraviolet light incident on the diffusion transmission member 5 travels in the diffusion transmission member 5 while being repeatedly refracted or reflected and is emitted outward when the ultraviolet light reaches a principal surface 5a of the diffusion transmission member 5. In this way, the ultraviolet light is diffused and transmitted through the diffusion transmission member 5. In Fig. 5, from the viewpoint of avoidance of complexity, illustrations of behavior of the reflected or refracted ultraviolet light inside the diffusion transmission member 5 are omitted.

Regarding ultraviolet light within a wavelength band of 190 nm or more and less than 240 nm, the diffusion transmission member 5 should have a ratio of the integral value of the outgoing ultraviolet light intensity to the light intensity of the incident ultraviolet light (hereinafter referred to as "average transmittance") of 1% or more so that the ultraviolet light generated by the light source 3 is extracted outside the housing 2. When the intensity of ultraviolet light that is generated by the light source 3 and within the wavelength range described above is satisfactorily high, the average transmittance of the diffusion transmission member 5 may fall below 10%.

The diffusion transmission member 5 may be a member that has a ceramic material such as silica, alumina, zirconia or yttria (Y₂O₃) as a main ingredient, for example, with proviso that ultraviolet light is diffused and transmitted through the member. More specifically, the diffusion transmission member is preferably a member made of a particle sintered body of silica or alumina in consideration of diffusional permeability to ultraviolet light. The diffusion transmission member 5 may be a filmy member made of a main ingredient such as PFA, PVDF or another fluorine-based resin, a polyethylene-based resin, or polycarbonate, polyethylene terephthalate (PET), or another polyester-based resin. Any of the resins displays diffusibility to ultraviolet light and thus is a preferable material for the diffusion transmission member 5.

In the first embodiment, the diffusion transmission member 5 has a thickness of 0.3 mm. The thickness of the diffusion transmission member 5 is preferably less than 1.5 mm, more preferably less than 1.0 mm, and particularly preferably less than 0.5 mm. In the present specification, the "thickness" of a member means a length of the member in the X direction.

With reference to Fig. 6, a reflection characteristic at a boundary surface 5b between the diffusion transmission member 5 and the optical filter 6 in the first embodiment will now be described. Assuming that ultraviolet light is isotropically diffused at a point P2 inside the diffusion transmission member 5, ultraviolet light L2, which reaches the point P2 after being transmitted through the optical filter 6 and incident on the diffusion transmission member 5, is diffused in various directions as indicated with one-dot chain lines in Fig. 6.

The ultraviolet light diffused at the point P2 includes ultraviolet light FL traveling to the +X side after being diffused at the point P2, ultraviolet light B0 traveling to the optical filter 6 side, i.e., the -X side, and being incident again on the optical filter 6, and ultraviolet light B1 being reflected off the boundary surface 5b between the diffusion transmission member 5 and the optical filter 6.

Fig. 7 is a graph showing an example of transmission spectra of the optical filter 6 by incident angle. The optical filter 6 is mostly characterized by having transmittance that inevitably changes according to the incident angle of the ultraviolet light and being more apt to reflect ultraviolet light incident at a greater angle. The characteristic is confirmed particularly with the optical filter 6 that is constituted by a dielectric multilayer film including a plurality of layers with different refractive indices. By such a characteristic, the ultraviolet light B0 and the ultraviolet light B1 as shown in Fig. 6 are generated.

As shown in Fig. 6, the diffusion transmission member 5 is disposed on an emission surface side of the optical filter 6. This enables the ultraviolet light B1 (return light) being refracted or reflected in the diffusion transmission member 5 and returning to the optical filter side (the -X side) to be reflected off the optical filter 6 so as to travel to the +X side and return again to the diffusion transmission member 5. This helps to increase the ultraviolet light FL traveling to an emission surface side of the diffusion transmission member 5 (the +X side) and improve usage efficiency of ultraviolet light emitted from the light source 3. Only part of the incident ultraviolet light is permitted to be transmitted through the diffusion transmission member 5, which is thus apt to decrease light intensity of the ultraviolet light. However, the diffusion transmission member disposed on the emission surface side of the optical filter 6 allows reuse of the return light and the light intensity of ultraviolet light emitted from the ultraviolet light irradiation device 1 to be increased.

In the first embodiment, the diffusion transmission member 5 may be disposed apart from the optical filter 6, with proviso that the diffusion transmission member is disposed on the emission surface side (the +X side) of the optical filter 6. However, from the viewpoint of increasing the return light, which is returned from the diffusion transmission member 5 and is reflected again off the optical filter 6, a distance between the optical filter 6 and the diffusion transmission member 5 is preferably as close as possible. Specifically, the distance between the optical filter 6 and the diffusion transmission member 5 is preferably 10 cm or less, more preferably 5 cm or less, and particularly preferably 1 cm or less. As shown in Fig. 6, it is desirable that the optical filter 6 and the diffusion transmission member 5 are disposed so as to be put into contact with each other.

### (Verification experiment)

A verification experiment was conducted to ascertain how a ratio (hereinafter referred to as a "target light ratio") between the average transmittance to ultraviolet light in the wavelength band used for inactivation (hereinafter referred to as "target light" or "safe light") and the average transmittance to ultraviolet light in the wavelength band hazardous to the human body (hereinafter referred to as "harmful light" changes in response to a change in thickness of the diffusion transmission member 5, and details of the experiment will be described below. The diffusion transmission member 5 used for this verification was a PTFE sheet, which is also used as the diffusion transmission member 5 in the first embodiment.

The target light was ultraviolet light having a wavelength within a range of 200 nm to 235 nm, and the harmful light was ultraviolet light having a wavelength within a range of 240 nm to 300 nm, in consideration of distinguishing clearly between the effect of light intensity attenuation by the optical filter 6, and the target light and the harmful light.

Thicknesses of the diffusion transmission member 5 subject to the verification were 0.05 mm, 0.1 mm, 0.25 mm, 0.5 mm, 1 mm, 1.5 mm, 2 mm, 2.5 mm, and 3 mm.

Intensities of ultraviolet light, i.e., ultraviolet light that was transmitted through the diffusion transmission member 5 (first light) and ultraviolet light that was not transmitted through the diffusion transmission member 5 (second light), were measured using a spectrophotometer and an integrating sphere. Specifically, with a combination of a spectrophotometer (V-7200) made by JASCO Corporation and an integrating sphere unit (Φ60mm integrating sphere unit) that is an accessory thereof, a value of integrated intensities of ultraviolet light emitted from a light source and incident on the spectrophotometer was measured under each condition. Intensities of the first light were measured by getting ultraviolet light emitted from the light source to be incident on the diffusion transmission member 5 and the ultraviolet light transmitted through the diffusion transmission member 5 to be incident on the integrating sphere unit. Intensities of the second light were measured by getting ultraviolet light emitted from the light source to be directly incident on the integrating sphere unit after the diffusion transmission member 5 was removed from a measurement system for the first light.

The average transmittance was calculated by dividing the value of integrated intensities of the second light by the value of integrated intensities of the first light, which were measured through the measurement method above.

### (Results)

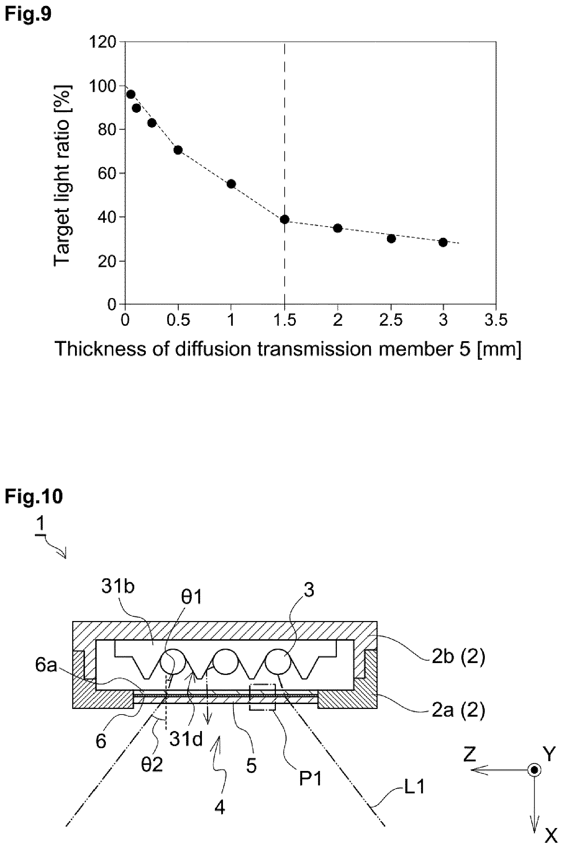

Fig. 8 is a graph showing a relationship between the thickness of the diffusion transmission member 5 and the average transmittance. Fig. 9 is a graph showing a relationship between the thickness of the diffusion transmission member 5 and the ratio of the average transmittance to the target light to the average transmittance to the harmful light (a target light ratio) and is a graph obtained by dividing each of the average transmittance to the target light by the average transmittance to the harmful light for each of the thicknesses of the diffusion transmission member 5, shown in Fig. 8.

In the graph shown in Fig. 8, the vertical axis represents the average transmittance and the horizontal axis represents the thickness of the diffusion transmission member 5. In the graph shown in Fig. 9, the vertical axis represents the target light ratio, and the horizontal axis represents the thickness of the diffusion transmission member 5.

As shown in Fig. 8, it is proved that the average transmittance to ultraviolet light decreases with an increase in thickness of the diffusion transmission member 5, and the average transmittance to the target light tends to decrease to a greater extent compared with the harmful light. It is also proved that the average transmittance greatly improves when the thickness of the diffusion transmission member 5 is less than 1.5 mm.

As shown in Fig. 9, it is proved that the target light ratio gets lower with an increase in thickness of the diffusion transmission member 5. This indicates that out of ultraviolet light emitted from the diffusion transmission member 5, the harmful light is relatively apt to be emitted and the target light is less apt to be emitted with an increase in thickness of the diffusion transmission member 5.

As shown in Fig. 9, the target light ratio can be improved to 50% or higher when the thickness of the diffusion transmission member 5 is in a range of 1 mm or less. Moreover, the target light ratio can be improved to around 70% when the thickness of the diffusion transmission member 5 is 0.5 mm or less, and the target light ratio can be improved to around 80% when the thickness of the diffusion transmission member 5 is 0.25 mm or less.

It is proved that there is a change in tendency when the thickness of the diffusion transmission member 5 is particularly 0.5 mm and 1.5 mm. It is inferred that such a change occurred because the target light was perfectly diffused when the thickness of the diffusion transmission member 5 was close to 0.5 mm and the harmful light was perfectly diffused when the thickness of the diffusion transmission member 5 was close to 1.5 mm.

This is because ultraviolet light is more apt to be refracted (dispersed) inside the diffusion transmission member 5 with an increase in diffusion transmission member thickness, as described above, and when the incident ultraviolet light is in a band of shorter wavelengths, the ultraviolet light is more apt to be refracted (dispersed). More specifically, with a decrease in wavelength of the incident ultraviolet light, a threshold value of the thickness of the diffusion transmission member 5 at which the light is perfectly diffused decreases. When the thickness of the diffusion transmission member 5 is greater than or equal to the threshold value, the diffusibility remains unchanged, and the energy of ultraviolet light is more apt to be consumed inside the diffusion transmission member 5 with an increase in diffusion transmission member thickness. On the other hand, with an increase in wavelength of the incident ultraviolet light, a threshold value of the thickness of the diffusion transmission member 5 at which the light is perfectly diffused rises. It is considered that a difference between the threshold values of the thickness of the diffusion transmission member 5 at which the ultraviolet light is perfectly diffused affects changes in target light ratio.

When the thickness of the diffusion transmission member 5 exceeds 1.5 mm and is at least in a range of 3.5 mm or less, the average transmittance to both the target light and the harmful light decreases with an increase in thickness of the diffusion transmission member 5. At this time, the average transmittance to the target light decreases to a greater extent compared with the harmful light. This can be proven by the target light ratio monotonically decreasing with an increase in thickness of the diffusion transmission member 5 in Fig. 9.

In the ultraviolet light irradiation device 1, the harmful light is substantially attenuated by the optical filter 6. However, if the diffusion transmission member 5 is thick, the target light is apt to be attenuated, and the relative percentage of the harmful light increases even slightly. This compromises safety desired when ultraviolet light is used for a long time. Thus, it is preferable to use a range in which the diffusion transmission member diffuses the target light and concurrently displays satisfactory transparency. Therefore, as described above, the thickness of the diffusion transmission member 5 is preferably less than 1.5 mm, more preferably less than 1.0 mm, and particularly preferably less than 0.5 mm. It should be noted that the thickness of the diffusion transmission member 5 is preferably 0.01 mm or more to produce an effect of ultraviolet light diffusion.

The ultraviolet light irradiation device 1 configured as described above is able to irradiate a wider area with ultraviolet light having a wavelength within a range of 190 nm or more and less than 240 nm, which has an extremely small influence on the human body, while suppressing a decrease in average transmittance to the ultraviolet light in the wavelength band and ensuring safety.

### [Second embodiment]

A description will be given to a configuration of the ultraviolet light irradiation device 1 according to a second embodiment of the present invention, mainly focusing on differences from the first embodiment.

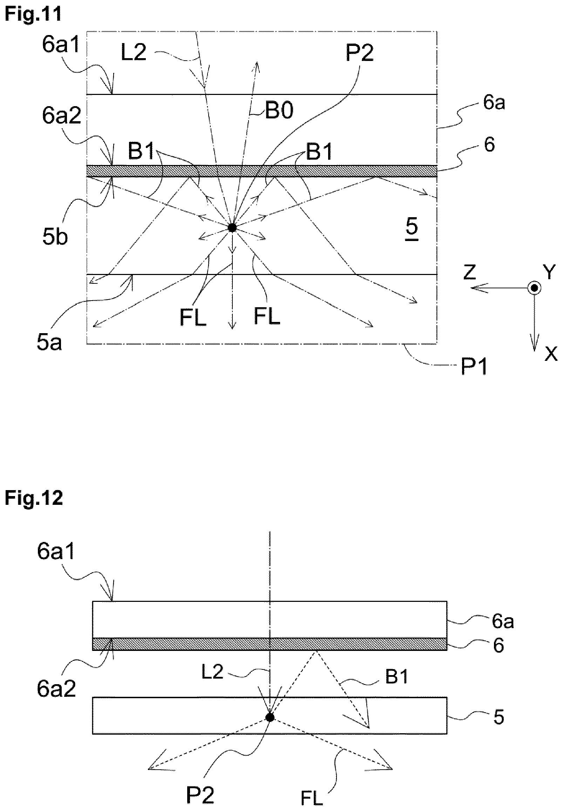

Fig. 10 is a cross-sectional view of the ultraviolet light irradiation device 1 according to the second embodiment, viewed along the Y direction. Fig. 11 is an enlarged view of a region P1 in Fig. 10. As shown in Figs. 10 and 11, the light extraction part 4 includes a base material 6a, the optical filter 6 disposed on a second principal surface 6a2 of the base material 6a, and the diffusion transmission member 5 disposed so as to be put into contact with the optical filter 6.

In the second embodiment, ultraviolet light is incident on a first principal surface 6a1 of the base material 6a, and superimposition of the base material 6a, the optical filter 6 formed on the second principal surface 6a2 of the base material 6a, and the diffusion transmission member 5 forms the light extraction part 4. In other words, ultraviolet light emitted from the light source 3 passes through the base material 6a, the optical filter 6 and the diffusion transmission member 5 forming the light extraction part 4 and is emitted as ultraviolet light L1 out of the housing 2. On the second principal surface 6a2 of the base material 6a, the optical filter 6 is formed and an additional layer such as a protection film may be formed to protect the optical filter 6. Each of the layers or films is formed so as to be thinner than the base material 6a.

In the second embodiment, the base material 6a is a member having a plate shape and being made of quartz glass. The base material 6a may be made of a material, such as borosilicate glass, other than quartz glass with proviso that the material can transmit ultraviolet light emitted from the light source 3.

With reference to Fig. 11, a reflection characteristic at the boundary surface 5b between the diffusion transmission member 5 and the optical filter 6 in the second embodiment will now be described, mainly focusing on differences from the configuration in the first embodiment. Assuming that ultraviolet light is isotropically diffused at a point P2 inside the diffusion transmission member 5, ultraviolet light L2, which reaches the point P2 after being transmitted through the optical filter 6 and incident on the diffusion transmission member 5, is diffused in various directions as indicated with one-dot chain lines in Fig. 11.

As shown in Fig. 11, ultraviolet light B1 being refracted or reflected in the diffusion transmission member 5 and returning to the optical filter side (the -X side) is reflected off the optical filter 6 so as to travel to the +X side without entering into the base material 6a. The ultraviolet light B1 reflected off the optical filter 6 is emitted from the principal surface 5a of the diffusion transmission member 5 unless the light is diffused again to the -X side.

After being transmitted through the optical filter 6, ultraviolet light B0 passes through the base material 6a and travels into the housing 2. The ultraviolet light B0 traveling into the housing 2 is absorbed by a member inside the housing 2 and disappears unless the light is reflected off a member disposed in the housing 2 to travel again toward the base material 6a.

This configuration prevents the ultraviolet light B1 from moving back and forth inside the base material 6a and being absorbed by the base material 6a, and the intensity of the light is not attenuated. As a result, the ultraviolet light irradiation device 1 according to this embodiment is able to emit ultraviolet light with increased intensity from the light extraction part 4 compared with a case in which the optical filter 6 is disposed on the first principal surface 6a1 of the base material 6a.

A verification experiment was conducted to ascertain to what extent irradiance of ultraviolet light emitted from the ultraviolet light irradiation device 1 and radiated to a predetermined area differs between a case (reference example) in which the optical filter 6 is disposed on the first principal surface 6a1 of the base material 6a and a case (example) in which the optical filter is disposed on the second principal surface 6a2, and the verification experiment will be described below.

Fig. 12 is a schematic view showing a configuration of the example in the verification experiment. As shown in Fig. 12, the configuration of the example in which the optical filter 6 is disposed on the second principal surface 6a2 of the base material 6a is equivalent to that adopted for this embodiment.

A configuration of the reference example is equivalent to that of the example, as shown in Fig. 24, except that the optical filter 101 is disposed on the first principal surface 101a1 of the base material 101a.

Unlike the embodiment above, the verification experiment was conducted using a diffusion transmission member made of PTFE with a thickness of 0.5 mm for the convenience of member procurement.

The irradiance of ultraviolet light having a wavelength of 222 nm was measured at a place 5 cm apart from the light extraction part 4 in the X direction in both the reference example and the example to make a comparison. A result was obtained, showing that the irradiance in the example was about 9% higher than the irradiance in the reference example.

A comparison was also made under the similar conditions regarding irradiance maintenance factor, a ratio of irradiance the device had after being continuously lit relative to irradiance the device had immediately after the start of being lit. A result was obtained, showing that the irradiance maintenance factor in the example was about 5% higher than that in the reference example in terms of a ratio of irradiance the device had after being lit for 250 hours relative to irradiance the device had immediately after the start of being lit.

When quartz glass constituting the base material 6a is irradiated with ultraviolet light having a wavelength of about 200 nm, the quartz glass gradually deteriorates due to breakage of the silicon (Si) and oxygen (O) bond, and the transmittance to ultraviolet light decreases. In the reference example, as shown in Fig. 24, ultraviolet light moves back and forth inside the base material 6a put between the diffusion transmission member 5 and the optical filter 6. On the other hand, in the example, as shown in Fig. 12, ultraviolet light does not move back and forth inside the base material 6a. In other words, the amount of ultraviolet light traveling inside the base material 6a is large and the base material 6a is apt to deteriorate in the reference example compared with the example. Thus, it is inferred that the result described above was obtained because high transmittance was maintained over a long time in the example compared with the reference example. In general, if ultraviolet light has energy higher than the bond energy for a chemical bond present in a material irradiated with the ultraviolet light, the ultraviolet light can break the chemical bond. Thus, it is inferred that a similar result is obtained from a verification experiment on irradiance maintenance factor even if the base material 6a is made of a material other than quartz glass.

In other words, in the ultraviolet light irradiation device 1 configured as described above, the ultraviolet light B1 is reflected off the optical filter 6 and travels to the +X side without entering into the base material 6a. To put it another way, the ultraviolet light irradiation device 1 is able to emit an increased amount of the ultraviolet light B1, which is return light, with high intensity maintained. In other words, the ultraviolet light irradiation device 1 with improved light usage efficiency is achieved.

In addition, the configuration described above makes it possible to implement inactivation with ultraviolet light at high irradiance over a longer time.

### [Third embodiment]

A description will be given to a configuration of the ultraviolet light irradiation device 1 according to a third embodiment of the present invention, mainly focusing on differences from the first and the second embodiments.

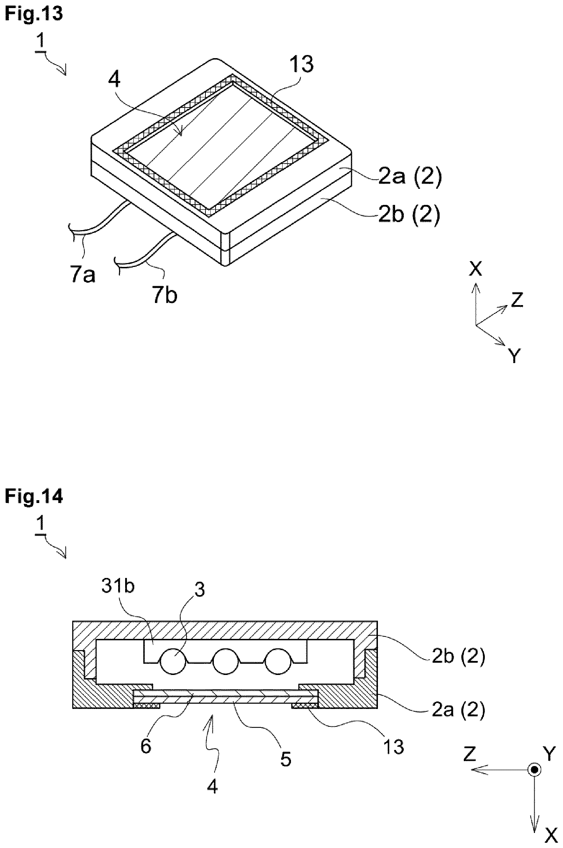

Fig. 13 is a schematic view showing an external appearance of the ultraviolet light irradiation device 1 according to a third embodiment, and Fig. 11 is a cross-sectional view of the ultraviolet light irradiation device 1 according to the third embodiment, viewed along the Y direction. Fig. 13 is a cross-sectional view of the ultraviolet light irradiation device 1 according to the third embodiment including the light extraction part 4 different in configuration from the light extraction part in Fig. 12, viewed along the Y direction. In the ultraviolet light irradiation device 1 according to the third embodiment, as shown in Figs. 10 to 12, the diffusion transmission member 5 and the optical filter 6 are fitted into a predetermined place on the cover member 2a, a component of the housing 2, and are fixed with a fastening member 13 to constitute the light extraction part 4. The fastening member 13 is disposed on an outer edge of the diffusion transmission member 5 and thus is able to fix a positional relationship between the diffusion transmission member 5 and the optical filter 6 without obstructing an optical path for ultraviolet light emitted from the light extraction part 4.

The configuration described above makes it possible to properly fix the diffusion transmission member 5 to an emission surface side of the optical filter 6 even if the diffusion transmission member is thin. The configuration makes it possible to support and stably fix the diffusion transmission member 5 with the fastening member 13 even if the diffusion transmission member is shaped like a sheet and is difficult to be fixed in isolation form. The diffusion transmission member 5 and the optical filter 6 may be detachable from the cover member 2a. This allows the diffusion transmission member 5 and the optical filter 6 to be replaced individually.

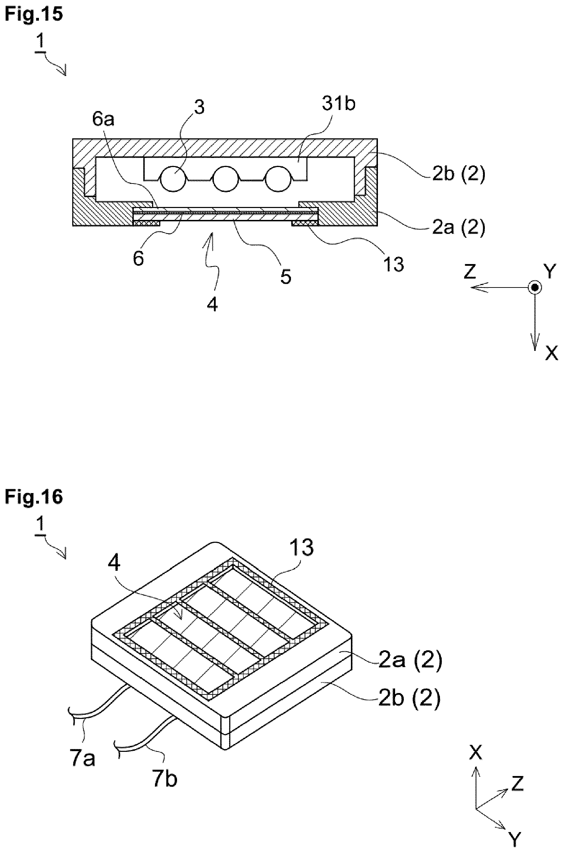

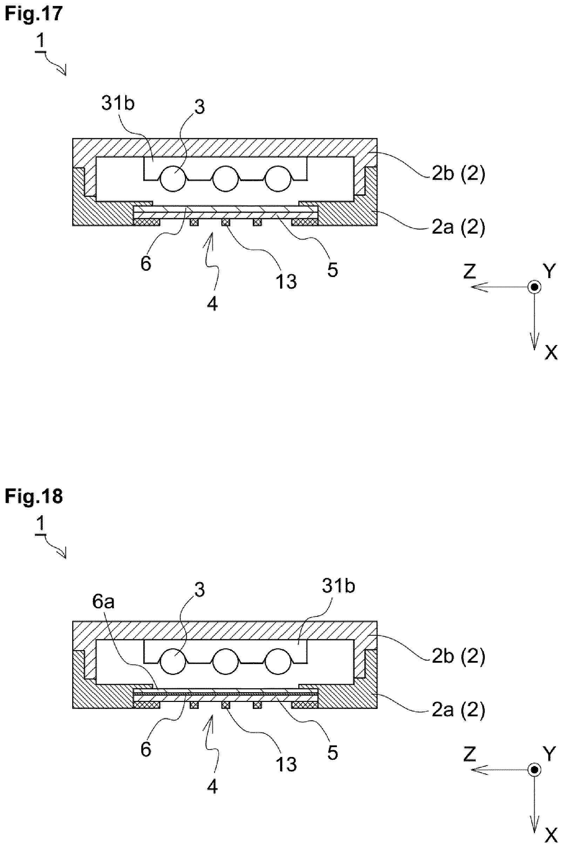

Fig. 16 is a schematic view showing an external appearance of the ultraviolet light irradiation device 1 according to the third embodiment different from the ultraviolet light irradiation device in Fig. 13, and Fig. 17 is a cross-sectional view of the ultraviolet light irradiation device 1 according to the third embodiment shown in Fig. 16, viewed along the Y direction. Fig. 18 is a cross-sectional view of the ultraviolet light irradiation device 1 according to the third embodiment including the light extraction part 4 different in configuration from the light extraction part in Fig. 17, viewed along the Y direction. The ultraviolet light irradiation device 1 according to the third embodiment, as shown in Figs. 12 and 13, may include the fastening member 13 that is structured so as to partly stretch over the light extraction part 4.

The configuration described above makes it possible to fix the diffusion transmission member 5 and the optical filter 6 with increased stability. The ultraviolet light irradiation device 1 configured as described above allows the diffusion transmission member 5 and the optical filter 6 to be replaced to suit a purpose such as intended use or a type of the light source 3.

Any of the above configurations are described in which the diffusion transmission member 5 and the optical filter 6 are allowed to be replaced together. However, the diffusion transmission member 5 and the optical filter 6 may be separable from each other such that these parts are allowed to be separately replaced. The diffusion transmission member 5 and the optical filter 6 may not be put into contact with each other.

### [Other embodiments]

### Other embodiments will now be described.

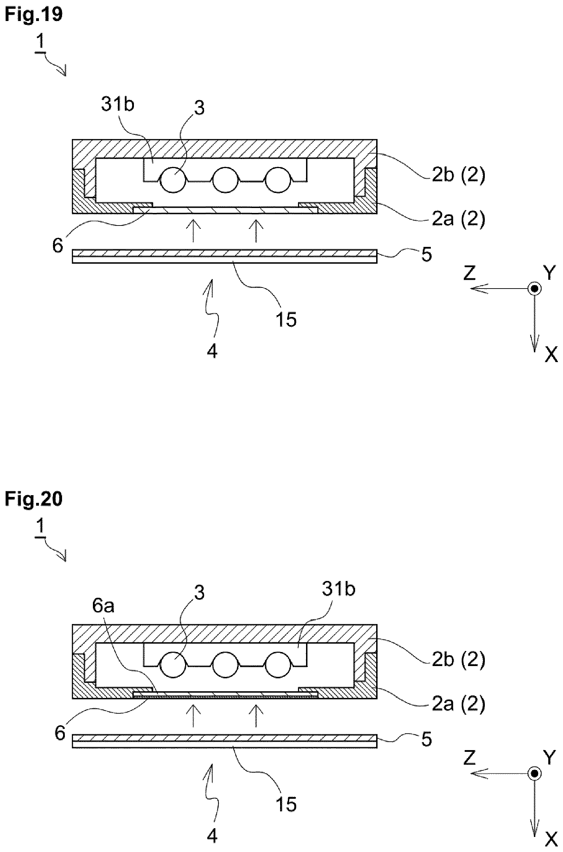

<1> Fig. 19 is a cross-sectional view of the ultraviolet light irradiation device 1 according to another embodiment, viewed along the Y direction. Fig. 20 is a cross-sectional view of the ultraviolet light irradiation device 1 according to another embodiment including the light extraction part 4 different in configuration from the light extraction part in Fig. 19, viewed along the Y direction. In the ultraviolet light irradiation device 1, as shown in Figs. 19 and 20, the diffusion transmission member 5 and a light transmission plate 15 that has, for example, quartz glass as a main ingredient and that displays high transparency to ultraviolet light may be superimposed on the optical filter 6 formed at the cover member 2a to constitute the light extraction part 4. According to the configuration, the light transmission plate 15 functions as a fastening member and makes it possible to fix the diffusion transmission member 5 that has a sheet, film, thin film, or other shape to the emission surface side of the optical filter 6 with increased properness. In the third embodiment, the optical filter 6 and the diffusion transmission member 5 may be separated from each other. The diffusion transmission member 5 and the light transmission plate 15 may be fixed, for example, with an adhesive. However, ultraviolet light passing through the optical filter 6 and the diffusion transmission member 5 is absorbed by the adhesive and the emitted ultraviolet light is apt to be attenuated, and it is possible that the adhesive itself is degraded by the ultraviolet light. Thus, the adhesive is desirably formed only partly between the optical filter 6 and the diffusion transmission member 5 such that an optical path that ultraviolet light is allowed to pass through without through the adhesive is formed. In addition, the adhesive is desirably formed only in a region that is not heavily irradiated with ultraviolet light (for example, a region where the light intensity is 50% or below, or more desirably 10% or below a peak intensity of the emitted light). In addition, it is desirable that the parts are mechanically fixed, for example, with a fixing tool without using an adhesive.

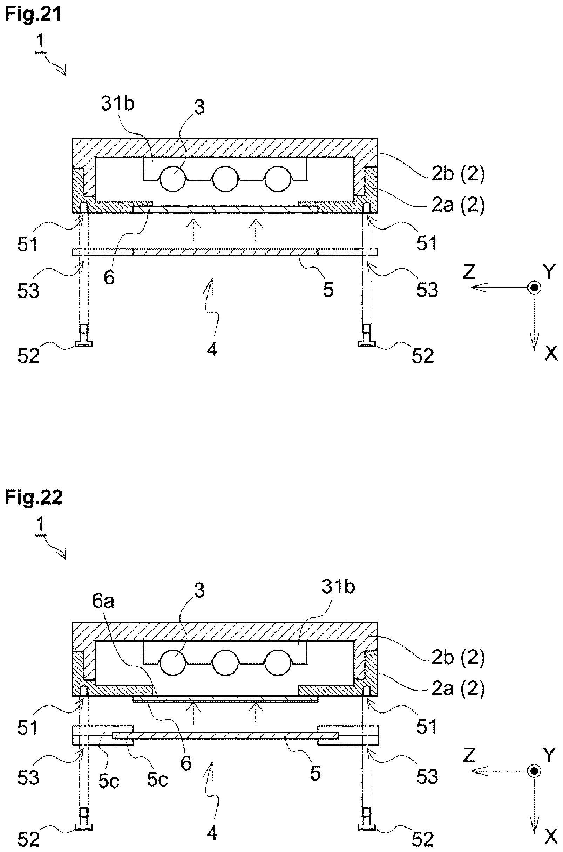

Fig. 21 is a cross-sectional view of the ultraviolet light irradiation device 1 according to another embodiment different from the ultraviolet light irradiation device in Fig. 19, viewed along the Y direction. Fig. 22 is a cross-sectional view of the ultraviolet light irradiation device 1 according to another embodiment including the light extraction part 4 different in configuration from the light extraction part in Fig. 21, viewed along the Y direction. In a configuration of the ultraviolet light irradiation device 1, as shown in Figs. 21 and 22, the diffusion transmission member 5 may be fixed to the cover member 2a with screws 52 that are inserted through through-holes 53 formed in the diffusion transmission member 5 and that are tightened into respective screw holes 51 formed in the cover member 2a.

The configuration described above makes it possible to fix the diffusion transmission member 5 and the optical filter 6 with increased stability. The ultraviolet light irradiation device 1 configured as described above allows the diffusion transmission member 5 and the optical filter 6 to be replaced to suit a purpose such as intended use or a type of the light source 3.

In a configuration of the ultraviolet light irradiation device 1, as shown in Fig. 22, the diffusion transmission member 5 may be fixed to the cover member 2a with screws 52 that are inserted through through-holes 53 formed in two plate-shaped holding members 5c clamping the diffusion transmission member 5 and that are tightened into respective screw holes 51 formed in the cover member 2a.

If the diffusion transmission member 5 is a member, such as a resin, a particle sintered body, or a sheet-shaped member, that is difficult to be fixed in isolation form to the housing 2, the holding members 5c clamps and holds the diffusion transmission member 5 to fix the diffusion transmission member to the housing 2.

In Fig. 22, the holding members 5c are illustrated as members holding a peripheral edge of the diffusion transmission member 5. However, the holding members may, for example, be a mesh-shaped plate member that has a plurality of holes through which ultraviolet light passes, and the diffusion transmission member 5 may be placed on a principal surface of the plate member. In the present embodiment, the two holding members 5c clamp the peripheral edge of the diffusion transmission member 5. However, the holding members 5c may be a single member.

When the plate-shaped holding members 5c are adopted, the holding members 5c are preferably made of a material that transmits ultraviolet light having a wavelength within a range of 190 nm or more and less than 240 nm to allow the ultraviolet light traveling toward the holding members 5c to be utilized even in a small degree for inactivation. The material that the holding members 5c are made of may be a material such as quartz glass or borosilicate glass.

<2> The ultraviolet light irradiation device 1 according to still another embodiment may include an optical element to improve directionality of ultraviolet light emitted from the light source and increase a component of light beams incident at a small angle on the optical filter 6. This makes it possible to improve light transmittance efficiency of the optical filter 6 by decreasing a component of light beams incident at a large angle on the optical filter 6 and increasing the component of light beams with a small incident angle. This is based on the characteristic of the optical filter 6 described above.

The optical element that may be used is an optical lens, an optical film, a reflecting member, or the like that is designed to improve the directionality of ultraviolet light emitted from the light source and control the angle of the light incident on the optical filter 6 to a small angle. When a light distribution angle of ultraviolet light emitted from the light source is considered, for example, the optical element is desirably disposed on an incident surface side of the optical filter 6 such that a range of angles in which the intensity of ultraviolet light emitted from the light source and incident on the optical filter 6 is 50% of the peak intensity is 50 degrees or less, 45 degrees or less, or more desirably 40 degrees or less. Further, the optical element may be disposed such that the range of angles is 35 degrees or less, or 30 degrees or less.

The above configuration allows ultraviolet light generated by the light source 3 to be extracted in an increased amount from the light extraction part 4. This helps to reduce power consumption and suppress a rise in temperature inside the housing and power source. This makes it possible to reduce the size and weight of the ultraviolet light irradiation device 1 or an overall system equipped with the ultraviolet light irradiation device 1.

<3> In the embodiments described above, the diffusion transmission member 5 and the optical filter 6 are disposed on the cover member 2a, which is a component of the housing 2. However, these parts may be mounted on a member other than the housing 2 and may be mounted, for example, on a wall surface of the light-emitting tube 30 included in the light source 3.

In the ultraviolet light irradiation device 1, the diffusion transmission member 5 may be made thicker rather than being made thinner. Specifically, the thickness of the diffusion transmission member 5 may be 0.5 mm or more, or greater than 0.5 mm. This configuration can be applied to a case where the intensity of ultraviolet light emitted from the light source is required to be more attenuated when the device is used. While it is recommended to set the integrated irradiation dose of ultraviolet light to be within the regulation value (a tolerance limit value) as described above, there is a case where irradiation of an environment with ultraviolet light is required to be continued over a longer time to maintain inactivation in the environment. For instance, there is a conceivable case where a new virus is brought in an environment from the outside via a human or an aerosol even after viruses are inactivated in the environment.

Hence, the ultraviolet light irradiation device 1 configured as described above attenuates the light intensity of emitted ultraviolet light while suppressing uneven intensities of the ultraviolet light through the diffusion transmission member 5 to make it possible to irradiate an environment in which the ultraviolet light irradiation device is installed with the ultraviolet light over a longer time. This configuration is preferable in a case where it is difficult to control the intensity of ultraviolet light emitted from a light source to a weaker level through the light source. From the above viewpoint, the thickness of the diffusion transmission member 5 may be greater than 0.5 mm.

However, from the viewpoint of improvement of the usage efficiency of ultraviolet light and the target light ratio, the thickness of the diffusion transmission member 5 is desirably less than 1.5 mm. Thus, the diffusion transmission member 5 may be greater than 0.5 mm and less than 1.5 mm.

<4> Fig. 15 is a cross-sectional view of the ultraviolet light irradiation device 1 according to another embodiment, viewed along the Y direction. In the above embodiments, the light extraction part 4 is described as a window made up of the base material 6a on which the optical filter 6 is disposed and the diffusion transmission member 5 in a side surface of the housing 2. However, the light extraction part 4, as shown in Fig. 15, may be a simple opening through which ultraviolet light is allowed to pass. In this case, the base material 6a and the diffusion transmission member 5 may be disposed so as to cover the light extraction part 4 when the light extraction part 4 is viewed from the +X side. The base material 6a and the diffusion transmission member 5 may be disposed inside the housing 2.

However, from the viewpoint of increasing the return light, which is returned from the diffusion transmission member 5 and is reflected off the optical filter 6 to the +X side, and suppressing leakage of ultraviolet light that does not pass through the optical filter 6, the distance between the optical filter 6 and the diffusion transmission member 5 is preferably as close as possible. Specifically, the distance between the optical filter 6 and the diffusion transmission member 5 is preferably 10 cm or less, more preferably 5 cm or less, and particularly preferably 1 cm or less.

<5> In this embodiment, an excimer lamp is mounted as the light source 3. However, the light source 3 is not limited to excimer lamps, and a light source such as an LED may be adopted. An AIGaN-based LED or an MgZnO-based LED having a main emission wavelength of less than 240 nm can be adopted, for example.

In a case of using a coherent light source as the light source 3, the coherent light source may emit coherent ultraviolet light from a gas laser or a solid laser element, or the coherent light source may include a wavelength conversion element to newly generate coherent light having different wavelengths using light emitted from a gas laser or a solid laser element. The wavelength conversion element that may be used, for example, is a non-linear optical crystal that multiplies the frequency of light emitted from a laser element to generate high-order harmonic waves such as second harmonic generation (SHG) waves or third harmonic generation (THG) waves.

<6> In the present invention, as described above, the inventors have studied the use of ultraviolet light having a wavelength within a range of 190 nm or more and less than 240 nm, found that the ultraviolet light is attenuated and the target light ratio substantially changes due to the diffusion transmission member 5 in the wavelength band and considered a reason. This is distinguished from conventional techniques that have been actively studied for ultraviolet light or visible light in relatively long wavelength bands and will contribute to the efficient and safe use or utilization of ultraviolet light in the wavelength band discussed herein.

The configurations of the ultraviolet light irradiation device 1 described above are merely examples, and the present invention is not limited to the illustrated configurations.

### DESCRIPTION OF REFERENCE SIGNS

- 1: Ultraviolet light irradiation device
- 2: Housing
- 2a: Cover member
- 2b: Main body
- 3: Light source
- 4: Light extraction part
- 5: Diffusion transmission member
- 5a: Principal surface
- 5b: Boundary surface
- 5c: Holding member
- 6: Optical filter
- 6a: Base material
- 6a1: First principal surface
- 6a2: Second principal surface
- 7a, 7b: Feeder
- 8a, 8b: Feeding terminal
- 13: Fastening member
- 15: Light transmission plate
- 30: Light-emitting tube
- 31a,31b: Electrode
- 31c: First reflecting surface
- 31d: Tapered surface
- 32: Reflecting member
- 32a: Second reflecting surface
- 51: Screw hole
- 52: Screw
- 53: Through-hole
- 70: Photometer
- 100: Diffusion transmission member
- 101: Optical filter
- 101a: Base material
- 101a1: First principal surface
- 101a2: Second principal surface
- L1: Ultraviolet light
- θ: Incident angle
- θ1: Incident angle
- θ2: Emission angle

## Claims

1. An ultraviolet light irradiation device comprising:
a light source to emit ultraviolet light having a wavelength within a range of 190 nm or more and less than 240 nm;
a housing to house the light source;
a light extraction part to extract the ultraviolet light emitted from the light source out of the housing; and
a diffusion transmission member to diffuse and transmit the ultraviolet light.

2. The ultraviolet light irradiation device according to claim 1, wherein a thickness of the diffusion transmission member is less than 1.5 mm.

3. The ultraviolet light irradiation device according to claim 2, comprising an optical filter to transmit at least part of ultraviolet light having a wavelength within a range of 190 nm or more and less than 240 nm and suppress transmission of ultraviolet light having a wavelength within a range of 240 nm or more and less than 300 nm.

4. The ultraviolet light irradiation device according to claim 3, wherein ultraviolet light transmitted through the optical filter is incident on the diffusion transmission member.

5. The ultraviolet light irradiation device according to claim 1, comprising:
a base material having a plate shape and including a first principal surface disposed adjacent to the light source, the base material being configured to transmit ultraviolet light having a wavelength within a range of 190 nm or more and less than 240 nm; and
an optical filter disposed on a second principal surface of the base material opposite the first principal surface, the optical filter being configured to transmit at least part of ultraviolet light having a wavelength within a range of 190 nm or more and less than 240 nm and suppress transmission of ultraviolet light having a wavelength within a range of 240 nm or more and less than 300 nm,
wherein the diffusion transmission member diffuses and transmits ultraviolet light emitted from the optical filter.

6. The ultraviolet light irradiation device according to claim 5, wherein the diffusion transmission member and the optical filter are put into contact with each other.

7. The ultraviolet light irradiation device according to claim 5, comprising a holding member to hold the diffusion transmission member.

8. The ultraviolet light irradiation device according to claim 7, wherein the holding member transmits at least part of ultraviolet light having a wavelength within a range of 190 nm or more and less than 240 nm.

9. The ultraviolet light irradiation device according to claim 1, wherein the diffusion transmission member is detachable from the housing.

10. The ultraviolet light irradiation device according to claim 1, wherein a main ingredient of the diffusion transmission member is a fluorine-based resin, a polyethylene-based resin, or a polyester-based resin.

11. The ultraviolet light irradiation device according to claim 1, wherein a main ingredient of the diffusion transmission member is a ceramic material.

12. The ultraviolet light irradiation device according to claim 11, wherein the main ingredient of the diffusion transmission member is silica or alumina.

13. The ultraviolet light irradiation device according to any one of claims 10 to 12, wherein average transmittance of the diffusion transmission member to ultraviolet light having a wavelength within a range of 190 nm or more and less than 240 nm is 10% or more.
